# EUROPÄISCHE PATENTSCHRIFT

(11) **EP 1 279 670 B1**
(45) Veröffentlichungstag und Bekanntmachung des Hinweises auf die Patenterteilung: **13.10.2004**
(21) Anmeldenummer: 02015585.9
(22) Anmeldetag: 15.07.2002
(51) Int. Cl.: C07D 301/12, C07D 303/32, C07D 303/36, C07D 303/48, C07D 405/04

(54) **Polyaminosäure-katalysiertes Verfahren zur enantioselektiven Epoxidierung vom alpha,beta-ungesättigten Enonen und alpha,beta-ungesättigten Sulfonen**
Process for enantioselective epoxidation of alpha, beta-unsaturated enones and alpha, beta-unsaturated sulfones catalysed by polyamino acids
Procédé pour l'époxydation enantiosélective d'énones alpha, béta-insaturées et de sulfones alpha, beta-insaturées, catalysé par des acides polyaminés

(30) Priorität: 27.07.2001 DE 10136485
(43) Veröffentlichungstag der Anmeldung: 29.01.2003
(73) Patentinhaber: Bayer Chemicals AG, 51368 Leverkusen (DE)
(72) Erfinder: Geller, Thomas, Dr., 51373 Leverkusen (DE); Krüger, Christa Maria, Dr., 48149 Münster (DE); Militzer, Hans-Christian, Dr., 51519 Odenthal (DE)

(56) Entgegenhaltungen:
- EP-A- 0 403 252
- EP-A- 1 006 111
- EP-A- 1 006 127
- WO-A-96/33183
- ADGER B.M. ET AL: "Improved procedure for Julia-Colonna asymmetric epoxidation of alpha, beta-unsaturated ketones." JOURNAL OF THE CHEMICAL SOCIETY, PERKIN TRANSACTIONS 1., Nr. 23, - 7. Dezember 1997 (1997-12-07) Seiten 3501-3507, XP002219130 CHEMICAL SOCIETY. LETCHWORTH., GB ISSN: 0300-922X
- BAURES P.W. ET AL: "An efficient asymmetric synthesis of substituted phenyl glycidic esters" TETRAHEDRON LETTERS., Bd. 31, Nr. 45, 1990, Seiten 6501-6504, XP002006755 ELSEVIER SCIENCE PUBLISHERS, AMSTERDAM., NL ISSN: 0040-4039
- JULIA S. ET AL: "Synthetic enzymes. Part 2. Catalytic asymmetric epoxidation by means of polyamino-acids in a triphase system" JOURNAL OF THE CHEMICAL SOCIETY, PERKIN TRANSACTIONS 1., Nr. 6, 1982, Seiten 1317-1324, XP002006756 CHEMICAL SOCIETY. LETCHWORTH., GB ISSN: 0300-922X
- DHANDA, ANUPMA ET AL: "PaaSiCats: Novel polyamino acid catalysts" CHIRALITY (2000), 12(5/6), 313-317, XP008009815
- FLOOD R.W. ET AL: "Efficient asymmetric epoxidation of alpha, beta-unsaturated ketones using a soluble triblock polyethyleneglycol-polyamino acid catalyst" ORGANIC LETTERS., Bd. 3, Nr. 5, 8. März 2001 (2001-03-08), Seiten 683-686, XP002219131 ACS, WASHINGTON, DC., US ISSN: 1523-7060

## Beschreibung

Die Erfindung betrifft ein neues Verfahren zur enantioselektiven Epoxidierung von α,β-ungesättigten Enonen und α,β-ungesättigten Sulfonen unter Verwendung spezieller Polyaminosäuren als Katalysatoren und Phasentransferkatalysatoren als Co-Katalysatoren.

Chiral, nicht-racemische Epoxide sind als wertvolle Bausteine für die Herstellung von optisch aktiven Wirkstoffen und Materialien bekannt (z.B. a) *Bioorg. Med*. *Chem*., **1999,** *7*, 2145-2156; b) *Tetrahedron Lett.,* **1999,** *40,* 5421-5424). Diese Epoxide können durch enantioselektive Epoxidierung von Doppelbindungen hergestellt werden. Hierbei werden zwei Stereozentren in einem synthetischen Schritt aufgebaut. Es ist daher nicht überraschend, dass eine Vielzahl von Methoden entwickelt wurde, um Doppelbindungen enantioselektiv zu epoxidieren. Für neue, verbesserte Methoden zur enantioselektiven Epoxidierung besteht allerdings weiterhin ein großer Bedarf.

Unter den Epoxidierungsmethoden, die jeweils auf spezielle Substrate limitiert sind, finden sich auch Methoden zur enantioselektiven Epoxidierung von α,β-ungesättigten Enonen.

So wurde beispielsweise der Einsatz von chiral, nicht-racemischen Phasentransferkatalysatoren auf Alkaloidbasis zur Epoxidierung von Enonen in *Tetrahedron Lett.,* **1998,** *39,* 7563-7566, *Tetrahedron Lett.,* **1998,** *39,* 1599-1602 und *Tetrahedron Lett.,* **1976**, *21*, 1831-1834 beschrieben.

In *Tetrahedron Lett.,* **1998,** *39,* 7353-7356, *Tetrahedron Lett.,* **1998,** *39,* 7321-7322 und *Angew. Chem., Int. Ed. Engl.,* **1997,** *36,* 410-412 werden ferner Möglichkeiten zur metallkatalysierten asymmetrischen Epoxidierung von Enonen mittels organischer Hydroperoxide beschrieben.

In WO-A-99/52886 wird ferner beschrieben, dass eine enantioselektive Epoxidierung von Enonen in Gegenwart von Katalysatoren möglich ist, die auf Zuckern basieren. Eine weitere Methode zur Epoxidierung unter Verwendung von Zn-Organylen und Sauerstoff in Gegenwart eines Ephedrin-Derivats wurde in *Liebigs Ann.*/*Recueil,* **1997,** 1101-1113 veröffentlicht.

In *Angew. Chem., Int. Ed. Engl.,* **1980,** *19*, 929-930, *Tetrahedron,* **1984,** *40,* 5207-5211 und *J. Chem. Soc*., *Perkin Trans*. 1, 1982, 1317-24 wird die Epoxidierungsmethode von Juliä beschrieben, wonach enantiomeren- und diastereomerenangereicherte Polyaminosäuren in Gegenwart von wässriger Wasserstoffperoxid- und NaOH-Lösung sowie eines Aromaten bzw. halogenierten Kohlenwasserstoffs als Lösungsmittel in der Lage sind, die enantioselektive Epoxidierung von α,β-ungesättigten Enonen zu katalysieren. Weiterentwicklungen dieser sogenannten 3-phasigen-Bedingungen finden sich in *Org. Synth.; Mod. Trends, Proc. IUPAC Symp. 6th.,* **1986,** 275. Die Methode wird heute allgemein als Juliä-Colonna-Epoxidierung bezeichnet.

Gemäß EP-A-0 403 252 ist es möglich, bei dieser Juliä-Colonna-Epoxidierung an Stelle der ursprünglichen Lösungsmittel vorteilhafterweise auch aliphatische Kohlenwasserstoffe einzusetzen.

Die Epoxidierung unter 3-phasigen-Bedingungen hat weiterhin deutliche Nachteile. Die Reaktionszeiten liegen nach den Originalbedingungen selbst für reaktive Substrate im Bereich von Tagen. Für ein *trans*-Chalkon werden in Abhängigkeit von der verwendeten Polyaminosäure beispielsweise 1 - 6 Tage benötigt (*Tetrahedron,* **1984,** *40*, 5207-5211). Eine im Reaktionsgefäß ausgefiihrte Voraktivierung der Polyaminosäure, indem man für 12-48 h im Lösungsmittel unter Zusatz von NaOH-Lösung rührt, verkürzt die Reaktionszeit vieler Substrate auf 1 - 3 Tage. Hierbei ist keine Zwischenaufarbeitung des Katalysators erforderlich (EP-A-0 403 252). In Gegenwart des Systems NaOH/Wasserstoffperoxid kann die Voraktivierungszeit auf minimal 6 h reduziert werden (*J. Chem. Soc., Perkin Trans*. 1, **1995,** 1467-1468)

Trotz dieser Verbesserung kann die dreiphasige Methode nicht auf Substrate angewandt werden, welche gegen Hydroxid-Ionen empfindlich sind (*J. Chem. Soc., Perkin Trans*. 1, **1997,** 3501-3507). Ein weiterer Nachteil dieser klassischen Bedingungen besteht darin, dass die Polyaminosäure während der Reaktion (bzw. bereits während der Voraktivierung) ein Gel bildet. Dieses schränkt die erwünschte Durchmischung während der Reaktion ein und erschwert die Aufarbeitung des Reaktionsgemisches.

In WO-A-96/33183 wird als spezielle Ausführimgsform beschrieben, dass die enantioselektive Epoxidierung von Enonen auch in Gegenwart des Phasentransferkatalysators Aliquat® 336 ([(CH₃)(C₈H₁₇)₃ N⁺]Cl⁻) durchgeführt werden kann, wenn gleichzeitig eine Polyaminosäure, ein organisches Lösungsmittel wie z.B. Dichlormethan, in Wasser schwerlösliches Natriumperborat als Oxidationsmittel sowie Alkali (z.B. NaOH) zugegen sind. Über die Polyaminosäure wird in diesem Zusammenhang keine nähere Aussage gemacht.

In *Tetrahedron Lett.,* **2001,** 42, 3741-43 wird lediglich ganz allgemein beschrieben, dass bei der Epoxidierung des Phenyl-*E*-Styrylsulfons unter den klassischen 3-phasigen-Bedingungen als Phasentransferkatalysator (PTC) ebenfalls Aliquat 336 zugesetzt werden kann. Hierbei wird allerdings nur eine sehr geringe Reaktionsgeschwindigkeit (Reaktionszeit: 4 Tage) und ein schlechter Enantiomerenüberschuss (21% *ee*) erzielt. Jegliche weitere Angaben dazu, wie diese Reaktion durchgeführt wurde, fehlen.

Neben der ursprünglichen Juliá-Colonna-Epoxidierung unter dreiphasigen Bedingungen und den oben genannten Varianten wurden auch andere Reaktionsführungen entwickelt. Gemäß *Chem. Commun.,* **1997,** 739-740 können durch Verwendung von THF, 1,2 Dimethoxyethan, *tert*.-Butylmethylether oder Ethylacetat als Lösungsmittel, einer nicht-nucleophilen Base (z.B. DBU) und des Hamstoff-Wasserstoffperoxid-Komplexes als Oxidationsmittel (pseudo)-wasserfreie Reaktionsbedingungen verwirklicht werden. Unter diesen sogenannten 2-phasigen Reaktionsbedingungen erfolgt die Epoxidierung deutlich schneller. Auf diesem Wege ist daher gemäß *J. Chem. Soc., Perkin Trans*. 1, **1997,** 3501-3507 erstmals auch die enantioselektive Epoxidierung von Hydroxid-empfindlichen Enonen unter den Juliä-Colonna-Bedingungen möglich.

Als deutlicher Nachteil erweist sich jedoch die Beobachtung, dass die Polyaminosäure bei Verwendung der zweiphasigen Bedingungen in einem separaten Verfahren voraktiviert werden muß, um schnelle Reaktionszeiten und hohe Enantiomerenüberschüsse zu erzielen. Für diese Voraktivierung, die durch Rühren der Polyaminosäure in einer Toluol/NaOH-Lösung erfolgt, werden mehrere Tage benötigt. Gemäß *Tetrahedron Lett.,* **1998,** 39, 9297-9300 erhält man dann den benötigten voraktivierten Katalysator nach einer Wasch- und Trockenprozedur. Diese aktivierte Polyaminosäure bildet jedoch unter den 2-phasigen-Bedingungen eine Paste, welche die Durchmischung während der Reaktion sowie die nachfolgende Aufarbeitung erschwert. Gemäß EP-A-1 006 127 kann dieses Problem durch eine Adsorption der aktivierten Polyaminosäure an einen festen Träger gelöst werden. Auf Silicagel geträgerte Polyaminosäuren werden als SCAT (silica adsorbed catalysts) bezeichnet.

Ein weiterer Nachteil der bisherigen 2-phasigen-Bedingungen besteht darin, dass der Einsatz von teureren, nicht-nucleophilen Basen (z.B. DBU) notwendig ist, um die Reaktion zu ermöglichen.

Gemäß EP-A-1 006 111 besteht eine weitere Variante der Juliá-Colonna-Epoxidierung darin, dass die aktivierte Polyaminosäure in Gegenwart von Wasser, einem wassermischbaren Lösungsmittels (z.B. 1,2-Dimethoxyethan) und Natriumpercarbonat die enantioselektive Epoxidierung katalysiert. Aufgrund der Verwendung wassermischbarer Lösungsmittel gestaltet sich bei diesem Verfahren die Aufarbeitung (Extraktion) umständlich.

Bei der Juliá-Colonna-Epoxidierung hängt die Reaktionsgeschwindigkeit und der erreichbare Enantiomerenüberschuss (*ee*) stark von der verwendeten Polyaminosäure und der Art ihrer Herstellung ab (*Chirality*, **1997,** *9*, 198-202). Um etwa vergleichbare Ergebnisse zu erhalten, wird zur Entwicklung und Beschreibung neuer Methoden in der Literatur durchgängig ein Standardsystem mit Poly-L-leucin (pll) als Katalysator und *trans*-Chalkon als Edukt verwendet. Neben D- oder L-Polyleucin werden jedoch auch andere Polyaminosäuren wie beispielsweise D- oder L-Neopentylglycin mit Erfolg verwendet (EP-A- 1 006 127).

Die Aufgabe der vorliegenden Erfindung bestand darin, ein Verfahren bereitzustellen, welches die Polyaminosäure-katalysierte enantioselektive Epoxidierung von α,β-ungesättigten Enonen und α,β-ungesättigten Sulfonen ermöglicht, aber nicht den Nachteilen der oben beschriebenen Varianten der Juliä-Colonna-Epoxidierung unterliegt. Insbesondere sollte eine schnelle, breit anwendbare Methode gefunden werden, welche die Verwendung teurer Basen und Oxidationsmittel sowie potentiell problematische Arten der Reaktionsführung sowie der Aufarbeitung vermeidet. Gleichzeitig sollte das Verfahren Vorteile hinsichtlich der Raum/Zeit-Ausbeute, der Handhabbarkeit, der Ökonomie und Ökologie im technischen Maßstab bringen.

Überraschenderweise wurde nun gefunden, dass man die Epoxidierung von α,β-ungesättigten Enonen und α,β-ungesättigten Sulfonen unter dreiphasigen Bedingungen mit substantiell geringeren Reaktionszeiten bei teilweise deutlich erhöhten Enantiomerenüberschüssen durchführen kann, wenn die als Katalysator verwendete Polyaminosäure in Gegenwart eines Phasentransferkatalysators voraktiviert wird und im Anschluß daran die Epoxidierung in Gegenwart dieser voraktivierten Polyaminosäure sowie des Phasentransferkatalysators erfolgt.

Gegenstand der Erfindung ist somit ein Verfahren zur Epoxidierung von α,β-ungesättigten Enonen oder α,β-ungesättigten Sulfonen in Gegenwart
(1) einer wasserlöslichen Base,
(2) eines Oxidationsmittels,
(3) einer diastereomeren- und enantiomerenangereicherten Homo-Polyaminosäure als Katalysator,
(4) Wasser und
(5) eines mit Wasser nicht oder nur begrenzt mischbaren Lösungsmittels,
   dadurch gekennzeichnet, dass die Epoxidierung
(6) in Gegenwart eines Phasentransferkatalysators durchgeführt wird
und die Homo-Polyaminosäure vor der Epoxidierung in Gegenwart des Phasentransferkatalysators einer Voraktivierung unterworfen wird.

Entscheidend ist, dass das erfindungsgemäßen Verfahren in Gegenwart eines Phasentransferkatalysators durchgeführt wird: Verwendbar sind beispielsweise quartäre Ammoniumsalze, quartäre Phosphoniumsalze, Onium-Verbindungen oder Pyridiniumsalze.

Bewährt haben sich vor allem quartäre Ammonium- oder Phosphoniumsalze der allgemeinen Formel (I)

(R¹R²R³R⁴A)⁺X⁻ (I)

worin
- A: für N oder P steht,
- X⁻: für ein anorganisches oder organisches Anion steht,
- R¹ R², R³ und R⁴: gleich oder verschieden sind und für Alkyl-, Aryl-, Aralkyl-, Cycloalkyl- oder Heteroaryl-Reste stehen, die durch einen oder mehrere, gleiche oder verschiedene Halogenreste substituiert sein können, oder aber jeweils zwei Reste unter Einbindung von A einen C₄-C₆-Cycloalkyl-Ring bilden können.

Bewährt haben sich solche Phasentransferkatalysatoren der allgemeinen Formel (I), bei denen A und X⁻ die oben genannten Bedeutungen besitzen und R¹ , R², R³ und R⁴ gleich oder verschieden sind und für C₁-C₁₈-Alkyl, C₆-C₁₈-Aryl, C₇-C₁₉-Arakyl, C₅-C₇-Cycloalkyl oder C₃-C₁₈-Heteroaryl stehen.

Besonders geeignet sind ((C₄H₉)₄N)⁺Hal⁻, insbesondere ((C₄H₉)₄N)⁺Br⁻, ((C₄H₉)₄P)⁺Hal⁻, insbesondere ((C₄H₉)₄P)⁺Br⁻, ((C₄H₉)₄N)⁺HSO₄⁻, ((C₈H₁₇)₄N)⁺Br⁻, [(CH₃)(C₈H₁₇)₃N⁺]Cl⁻ und [(CH₃)(C₄H₉)₃ N⁺]Cl⁻ als Phasentransferkatalysatoren.

In der allgemeinen Formel (I) steht X für ein anorganisches oder organisches Kation, bevorzugt steht X für F⁻, Cl⁻, Br⁻, I⁻, OH⁻, HSO₄⁻, SO₄⁻, NO₃⁻, CH₃COO⁻, CF₃COO⁻, C₂H₅COO⁻, C₃H₇COO⁻, CF₃SO₃⁻ oder C₄F₉SO₃⁻

Die erfindungsgemäß einzusetzenden Phasentransferkatalysatoren sind üblicherweise käuflich erhältlich oder aber nach dem Fachmann geläufigen Methoden herstellbar.

Die Menge des zugesetzten Phasentransferkatalysators ist nicht kritisch und liegt üblicherweise im Bereich von 0.1 - 20 mol%, bevorzugt im Bereich von 0,5-15 mol%, besonders bevorzugt im Bereich von 0,5-11 mol%, jeweils bezogen auf das eingesetzte α,β-ungesättigte Enon oder α,β-ungesättigte Sulfon. Bei Mengen, die noch geringer sind als 0,1 mol%, ist jedoch zu beobachten, dass bei unverändert hohem Enantiomerenüberschuss die Reaktionsgeschwindigkeit deutlich abnimmt.

Als α,β-ungesättigte Enone oder α,β-ungesättigte Sulfone können Verbindungen der allgemeinen Formel (II) eingesetzt werden 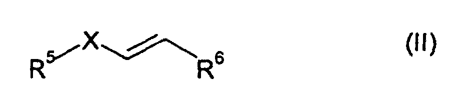 worin
- X: für (C=O) oder (SO₂) steht und
- R⁵ und R⁶: gleich oder verschieden sind und (C₁-C₁₈)-Alkyl, (C₂-C₁₈)-Alkenyl, (C₂-C₁₈)-Alkinyl, (C₃-C₈)-Cycloalkyl, (C₆-C₁₈)-Aryl, (C₇-C₁₉)-Aralkyl, (C₁-C₁₈)-Heteroaryl oder (C₂-C₁₉)-Heteroaralkyl bedeuten,
wobei die für R⁵ und R⁶ genannten Reste einfach oder mehrfach mit gleichen oder verschiedenen Resten R⁷, Halogen, NO₂, NR⁷R⁸, PO₀₋₃R⁷R⁸, SO₀₋₃R⁷, OR⁷, CO₂R⁷, CONHR⁷ oder COR⁷ substituiert sein können und gegebenenfalls eine oder mehrere CH₂-Gruppen in den Resten R⁵ und R⁶ durch O, SO₀₋₂, NR⁷ oder PO₀₋₂R⁷ substituiert sind,
wobei R⁷ und R⁸ gleich oder verschieden sind und H, (C₁-C₁₈)-Alkyl, (C₂-C₁₈)-Alkenyl, (C₂-C₁₈)-Alkinyl, (C₃-C₈)-Cycloalkyl, (C₆-C₁₈)-Aryl, (C₁-C₁₈)-Heteroaryl, (C₁-C₈)-Alkyl-(C₆-C₈)-Aryl, (C₁-C₈)-Alkyl-(C₁-C₁₉)-Heteroaryl, (C₁-C₈)-Alkyl-(C₃-C₈)-Cycloalkyl bedeuten und diese Reste R⁷ und R⁸ einfach oder mehrfach mit gleichen oder verschiedenen Halogenresten substituiert sein können.

Unter einem (C₁-C₁₈)-Alkylrest wird im Rahmen der Erfindung ein Rest mit 1 bis 18 gesättigten C-Atomen verstanden, der beliebige Verzweigungen aufweisen kann. *Insbesondere sind unter dieser Gruppe die Reste Methyl, Ethyl, *n*-Propyl, Isopropyl, *n*-Butyl, Isobutyl, *sec*-Butyl, *tert*-Butyl, Pentyl und Hexyl subsumierbar.

Ein (C₂-C₁₈)-Alkenylrest weist die für den (C₁-C₁₈)-Alkylrest genannten Merkmale auf, wobei innerhalb des Restes mindestens eine Doppelbindung vorhanden sein muss.

Ein (C₂-C₁₈)-Alkinylrest weist die für den (C₁-C₁₈)-Alkylrest genannten Merkmale auf, wobei innerhalb des Restes mindestens eine Dreifachbindung vorhanden sein muss.

Unter einem (C₃-C₈)-Cycloalkylrest wird ein cylischer Alkylrest mit 3 bis 8 C-Atomen und gegebenenfalls beliebiger Verzweigung verstanden. Insbesondere zählen hierzu Reste wie Cyclopropyl, Cyclobutyl, Cyclopentyl, Cyclohexyl und Cycloheptyl. In diesem Rest kann eine oder mehrere Doppelbindungen vorhanden sein.

Unter einem (C₆-C₁₈)-Arylrest wird ein aromatischer Rest mit 6 bis 18 C-Atomen verstanden. Insbesondere zählen hierzu Reste wie Phenyl-, Naphthyl-, Anthryl- und Phenanthryl.

Unter einem (C₇-C₁₉)-Aralkylrest wird ein über einen (C₁-C₈)-Alkylrest an das Molekül gebundener (C₆-C₁₈)-Arylrest verstanden.

Ein (C₁-C₁₈)-Heteroarylrest bezeichnet im Rahmen der Erfindung ein fünf-, sechsoder siebengliedriges aromatisches Ringsystem mit 1 bis 18 C-Atomen, welches ein oder mehrere Heteroatome, bevorzugt N, O oder S im Ring aufweist. Zu diesen Heteroarylresten zählen z.B. 1-, 2-, 3-Furyl, 1-, 2-, 3-Pyrrol, 1-, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, 1-, 3-, 4-, 5-Triazolyl, 1-, 4-, 5-Tetrazolyl, Acridinyl, Chinolinyl, Phenanthridinyl, 2-, 4-, 5-, 6-Pyrimidinyl und 4-, 5-, 6-, 7-(1-Aza)-indolizinyl.

Unter einem (C₂-C₁₉)-Heteroaralkylrest wird ein dem (C₇-C₁₉)-Aralkylrest entsprechendes heteroaromatisches System verstanden.

Unter Halogen oder auch Hal versteht man im Kontext dieser Erfindung Fluor, Chlor, Brom und Iod.

Als Substrate des erfindungsgemäßen Verfahrens werden bevorzugt α,β-ungesättigte Enone oder α,β-ungesättigte Sulfone der allgemeinen Formel (II) eingesetzt, in denen
- R⁵ und R⁶: gleich oder verschieden sind und (C₁-C₁₂)-Alkyl, (C₂-C₁₂)-Alkenyl, (C₂-C₁₂)-Alkinyl, (C₅-C₈)-Cycloalkyl, (C₆-C₁₂)-Aryl oder (C₁-C₁₂)-Heteroaryl bedeuten, wobei die zuvor genannten Reste einfach oder mehrfach mit gleichen oder verschiedenen Resten R⁷, Halogen, NO₂, NR⁷R⁸, PO₀₋₃R⁷R⁸ oder OR⁷ substituiert sein können und R⁷ und R⁸ die für die allgemeine Formel (II) zuvor angegebene Bedeutungen besitzen.

Besonders bevorzugt werden als Substrate des erfindungsgemäßen Verfahrens α,β-ungesättigte Enone oder α,β-ungesättigte Sulfone der allgemeinen Formel (II) eingesetzt, in denen
- R⁵ und R⁶: gleich oder verschieden sind und (C₁-C₁₂)-Alkyl, (C₂-C₁₂)-Alkenyl, (C₂-C₁₂)-Alkinyl, (C₅-C₈)-Cycloalkyl, (C₆-C₁₂)-Aryl oder (C₁-C₁₂)-Heteroaryl bedeuten, wobei die zuvor genannten Reste einfach oder mehrfach mit gleichen oder verschiedenen Resten R⁷, Halogen, NO₂, NR⁷R⁸, PO₀₋₃R⁷R⁸ oder OR⁷ substituiert sein können und R⁷ und R⁸ die für die allgemeine Formel (II) zuvor angegebene Bedeutungen besitzen,
mit der Maßgabe, dass mindestens einer der Reste R⁵ oder R⁶ einen (C₂-C₁₂)-Alkenyl-, (C₂-C₁₂)-Alkinyl-, (C₆-C₁₂)-Aryl- oder (C₁-C₁₂)-Heteroarylrest darstellt.

Insbesondere ist es bevorzugt, Substrate der allgemeinen Formel (III) der erfindungsgemäßen Epoxidierung zu unterwerfen: 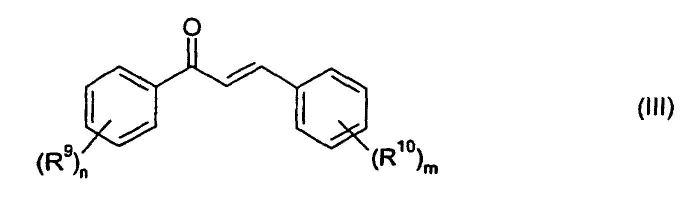 wobei
- n und m: gleich oder verschieden sind und für die Zahlen 0, 1, 2 oder 3 stehen,
und
- R⁹ und R¹⁰: gleich oder verschieden sind und NR⁷R⁸, NO₂, OR⁷, (C₁-C₁₂)-Alkyl, (C₂-C₁₂)-Alkenyl, (C₂-C₁₂-Alkinyl, (C₅-C₈)-Cycloalkyl, (C₆-C₁₂)-Aryl oder (C₁-C₁₂)-Heteroaryl bedeuten, wobei diese Reste R⁹ und R¹⁰ einfach oder mehrfach mit gleichen oder verschiedenen Halogenresten substituiert sein können und R ⁷ und R⁸ die zuvor für Formel (II) genannten Bedeutungen besitzen.

Das erfindungsgemäße Verfahren zur Herstellung der enantiomerenangereicherten Epoxide wird in Gegenwart von Homo-Polyaminosäuren als Katalysator durchgeführt, die zuvor in Gegenwart des Phasentransferkatalysators einer Voraktivierung unterzogen wurden.

Für die Voraktivierung können die verschiedensten diastereomeren- und enantiomerenangereicherten Homo-Polyaminosäuren zum Einsatz kommen. Vorzugsweise werden allerdings Homo-Polyaminosäuren aus der Gruppe Polyneopentylglycin, Polyleucin, Polyisoleucin, Polyvalin und Polyalanin sowie Polyphenylalanin benutzt. Aus dieser Gruppe ist Polyneopentylglycin und Polyleucin am meisten zu bevorzugen.

Die Kettenlänge der Polyaminosäuren ist so zu wählen, dass einerseits die chirale Induktion bei der Reaktion nicht beeinträchtigt wird und andererseits die Kosten für die Synthese der Polyaminosäuren nicht zu groß werden. Vorzugsweise liegt die Kettenlänge der Homo-Polyaminosäuren zwischen 5 und 100, vorzugsweise 7 bis 50, Aminosäuren. Ganz besonders bevorzugt ist eine Kettenlänge von 10 bis 40 Aminosäuren.

Die Homo-Polyaminosäuren können entweder als solche unverändert in die Voraktivierung eingesetzt werden oder aber zuvor mit polyfunktionellen Aminen vernetzt oder durch andere organische Polymere kettenverlängert werden. Für eine Vernetzung setzt man vorteilhafterweise als vernetzende Amine Diaminoalkane, bevorzugt 1,3-Diaminopropan, oder quervemetztes Hydroxy- oder Aminopolystyrol (CLAMPS, kommerziell erhältlich) ein. Als Polymervergrößerer kommen bevorzugt auf Polyethylenglykol oder Polystyrol basierende Nucleophile in Frage. Derart veränderte Polyaminosäuren sind in *Chem. Commun.,* **1998,** 1159-1160 und *Tetrahedron: Asymmetry,* **1997,** *8*, 3163-3173 dargestellt.

Für den Einsatz im erfindungsgemäßen Verfahren werden die Homo-Polyaminosäuren einer Voraktivierung in Gegenwart eines Phasentransferkatalysators unterzogen. Hierzu werden die Polyaminosäure, der Phasentransferkatalysator sowie üblicherweise das Oxidationsmittel und die Base im Lösungsmittel suspendiert und für eine Zeit im Bereich von 15 Minuten bis 2 Stunden gerührt. Nach dieser Voraktivierung kann die voraktivierte Homo-Polyaminosäure entweder zwischenisoliert werden, oder das Reaktionssystem kann unmittelbar in der nachfolgenden Epoxidierung eingesetzt werden. Bei der Voraktivierung entsprechen die og. Komponenten, Base und Oxidationsmittel, den für die nachfolgende Epoxidierungsreaktion beschriebenen.

Die zur Voraktivierung einzusetzenden Homo-Polyaminosäuren können nach Methoden des Standes der Technik hergestellt werden (*J. Org. Chem.* 1993, 58, 6247 und *Chirality* 1997, 9, 198-202). Die Methode ist auf beide optischen Antipoden der Aminosäuren anzuwenden. Der Einsatz einer bestimmten Antipode einer Polyaminosäure korreliert mit der Stereochemie des Epoxids, d.h. eine Poly-L-aminosäure führt zur optischen Antipode des Epoxids, das mit einer Poly-D-aminosäure erhalten wird.

Die Menge der eingesetzten Homo-Polyaminosäure ist nicht kritisch und liegt üblicherweise im Bereich von 0.0001 - 40 mol%, bevorzugt im Bereich von 0,001-20 mol%, besonders bevorzugt im Bereich von 0,01-15 mol%, und insbesondere 1 bis 15 mol-%, jeweils bezogen auf das eingesetzte α,β-ungesättigte Enon oder α,β-ungesättigte Sulfon.

Als Oxidationsmittel dienen in der Regel Peroxide, Persäuren oder anorganische Oxidationsmittel wie Natriumhypochlorit oder Natriumpercarbonat. Bevorzugt sind Peroxide, Persäuren oder Natriumhypochlorit. Besonders bevorzugt wird eine wässrige H₂O₂-Lösung eingesetzt. Diese wässrige Lösung kann dabei alle üblichen Konzentrationen haben. Weitere bei dieser Reaktion einzusetzende Oxidationsmittel sind die in *Methoden Org. Chem. (Houben-Weyl),* Band 4/1a+b, 59-319 sowie die in *Oxidation in Organic Chemistry,* ACS Monograph 186, Washington DC, **1990,** 1-47 genannten Verbindungen.

Die Menge des eingesetzten Oxidationsmittels kann in breiten Grenzen von 1 - 40 Äquivalenten variiert werden. Überraschender- und vorteilhafterweise gelingt die erfindungsgemäße Umsetzung bei weiterhin niedrigen Reaktionszeiten und hohen Enantiomerenüberschüssen auch mit relativ geringen Mengen Oxidationsmittel im Bereich von 1 - 10 Äquivalenten, bevorzugt von 1-3 Äquivalenten, besonders bevorzugt von 1,1 - 2,5 Äquivalenten.

Das erfindungsgemäße Verfahren wird in Gegenwart einer wasserlöslichen Base durchgeführt. Bewährt hat sich hierbei der Einsatz von Alkalimetallhydroxiden, wie NaOH, KOH oder LiOH. Die Base wird üblicherweise in Form einer wässrigen Lösung eingesetzt.

Die Menge der eingesetzten Base kann in breiten Grenzen von 0,1-10 Äquivalenten variiert werden. Überraschender- und vorteilhafterweise gelingt die erfindungsgemäße Umsetzung bei weiterhin niedrigen Reaktionszeiten und hohen Enantiomerenüberschüssen auch mit relativ geringen Mengen Base im Bereich von 0,5-5 Äquivalenten, bevorzugt 0,8-2 Äquivalenten.

Das erfindungsgemäße Verfahren wird unter Einsatz eines nicht oder nur begrenzt mit Wasser mischbaren Lösungsmittels durchgeführt. Als begrenzt mit Wasser mischbar wird ein Lösungsmittel im Kontext dieser Erfindung erachtet, wenn eine Mischung aus dem organischen Lösungsmittel und Wasser bei 20°C nicht mehr als 20 Gew.%, bevorzugt nicht mehr als 10 Gew.% und insbesondere nicht mehr als 8 Gew.% Wasser enthalten kann, um einphasig zu bleiben.

Als organische Lösungsmittel kommen allgemein unsubstituierte oder substituierte aromatische Kohlenwasserstoffe, aliphatische Kohlenwasserstoffe, Halogenalkane sowie Ether in Betracht. Geeignet sind besonders Toluol, Xylol, Hexan, *tert.*-Butylmethylether, Diethylether, Chloroform und Methylenchlorid.

Gefunden wurde, dass die Homo-Polyaminosäure pll in *tert*.-Butylmethylether aggregiert. Für eine kontinuierliche Reaktionsführung ist daher der *tert*.-Butylmethylether ein interessantes und geeignetes Lösungsmittel.

Die Temperatur, die bei der Epoxidierung verwendet wird, liegt im allgemeinen im Bereich von -10 bis +50 °C, bevorzugt im Bereich von 0 bis +40 °C und insbesondere bei +10 bis +30 °C.

Der pH-Wert, welcher während der Reaktion eingestellt wird, kann so gewählt werden, dass ein Überschuss an deprotoniertem H₂O₂ verglichen mit nicht deprotoniertem H₂O₂ vorhanden ist. Andererseits sollte der pH-Wert bei der Reaktion auch nicht so hoch gewählt werden, dass die eingesetzten organischen Verbindungen Schaden nehmen. Vorzugsweise liegt der pH-Wert im Bereich von 7 - 14, bevorzugt im Bereich von 7,5 - 13.

Der Wasseranteil des Systems resultiert üblicherweise daraus, dass wie bereits beschrieben einzelne Reaktionskomponenten des Systems, wie die Base und das Oxidationsmittel, in Form wässriger Lösungen eingesetzt werden. Insgesamt liegt der Wassergehalt des Reaktionsgemisches im Bereich von 1-70 Gew.%, bevorzugt im Bereich von 5-50 Gew.%, bezogen auf das gesamte Reaktionsgemisch.

Zur Voraktivierung der Homo-Polyaminosäure wird üblicherweise so verfahren, dass bis auf das zu epoxidierende Substrat alle Reaktionskomponenten des Verfahrens, d.h. Homo-Polyaminosäure, Phasentransferkatalysator, Base, Oxidationsmittel und Lösungsmittel zusammengegeben werden. Diese Mischung wird dann für einen Zeitraum von 15 Minuten bis 2 Stunden gerührt. Nach dieser Voraktivierung kann die voraktivierte Homo-Polyaminosäure entweder zwischenisoliert werden, oder aber bevorzugt kann das Reaktionssystem unmittelbar in der nachfolgenden Epoxidierung eingesetzt werden, d.h. das Substrat zugegeben werden. Im Falle der Zwischenisolierung wird die Homo-Polyaminosäure vom Reaktionsgemisch abgetrennt, beispielsweise durch Zentrifugieren, mit Wasser neutral gewaschen, mit Aceton gewaschen und getrocknet.

Das erfindungsgemäße Verfahren zeichnet sich durch stark verringerte Reaktionszeiten aus. Anstelle von Tagen reichen bereits wenige Stunden oder sogar nur Minuten aus, um die Epoxidierung der α,β-ungesättigten Enone und α,β-ungesättigten Sulfone mit hohem Umsatz und hoher Enantioselektivität zu erreichen.

Das erfindungsgemäße Verfahren vermeidet die bisher nach dem Stand der Technik erforderliche, sehr aufwendige Präparation der aktivierten Homo-Polyaminosäuren über einen Zeitraum von mehreren Tagen durch den Zusatz eines Phasentransferkatalysators. In Gegenwart eines solchen Phasentransferkatalysators gelingt die Aktivierung der Homo-Polyaminosäure innerhalb von Minuten bzw. maximal 2 h, während unter den ursprünglichen 3-phasigen-Bedingungen minimal 6 h bzw. unter den 2-phasigen-, den SCAT- und den Percarbonat-Bedingungen 4 - 5 Tage benötigt werden. Eine Zwischenisolierung der voraktivierten Homo-Polyaminosäure ist (im Gegensatz zu den 2-phasigen-, den SCAT- und den Percarbonat-Bedingungen) nicht notwendig.

Die erfindungsgemäße Verwendung des Phasentransferkatalysators als Co-Katalysator erlaubt es zusätzlich, die notwendige Menge des Oxidationsmittels sowie der Base deutlich zu reduzieren, ohne dass hierdurch Reaktionsgeschwindigkeit, Umsatz oder Enantiomerenüberschuss negativ beeinflußt werden. Zudem können besonders kostengünstige Basen und Oxidationsmittel eingesetzt werden.

Aufgrund der sehr kurzen Reaktionszeiten sind über das erfindungsgemäße Verfahren erstmals auch Hydroxid-empfindliche Substrate, die nach den klassischen 3-phasigen-Bedingungen (*JCS., Perkin Trans, 1*, **1997,** 3501-3507) nicht erfolgreich epoxidiert werden können, einer enantioselektiven Epoxidierung zugänglich.

### Beispiele:

Der Herstellungsprozess für Polyaminosäuren liefert oft Katalysatoren für die Juliä-Colonna Epoxidierung, die eine stark unterschiedliche katalytische Aktivität aufweisen (*Chirality*, **1997,** *9,* 198-202). Der Umsatz pro Zeiteinheit und der Enantiomerenüberschuss lassen sich für ein bestimmtes Substrat nur vergleichen, wenn für die Epoxidierungsreaktion dieselbe Polyaminosäure-Charge verwendet wird. Aus diesem Grund ist ein direkter Vergleich von neuen Ergebnissen mit in der Literatur publizierten Resultaten nicht möglich, da eben zwangsläufig unterschiedliche Katalysatorchargen verwendet werden. Aus diesem Grund wurden bei den nachfolgenden Beispielgruppen I-V jeweils einheitliche Polyleucin-Chargen verwendet (sowohl bei den erfindungsgemäßen Beispielen als auch bei den entsprechenden Vergleichsbeispiele).

In allen nachfolgenden Beispielen werden der Umsatz und der Enantiomerenüberschuss (*ee*-Wert) gemäß literaturbekannten Verfahren mittels HPLC an einer chiral, nicht-racemischen Phase bestimmt (UV-Detektion).

### Beispielgruppe I:

### Beispiele 1 und 2 sowie Vergleichsbeispiele VB 3 - 4

### Epoxidierung von trans-Chalkon (1) zu Epoxychalkon (2)

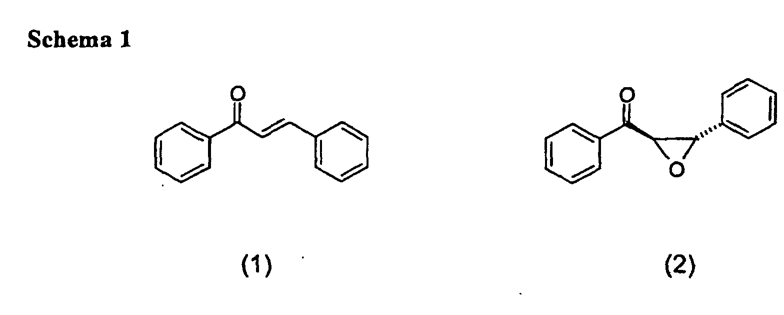

### Beispiele 1 und 2: 3-phasige Bedingungen mit PTC und mit pll-Voraktivierung

100 mg nicht voraktivierter pll sowie 8.5 mg (Bu₄N)⁺Br⁻ (Beispiel 1) bzw. 11 mg Aliquat 336 (Beispiel 2) wurden in einer Mischung aus 0.8 ml Toluol und 62 µl NaOH (eingesetzt als 5 molare wässrige Lösung, entspricht 1,3 Äquivalenten) suspendiert. Anschließend wurden 32µl H₂O₂ (eingesetzt als 30%ig wässrige Lösung, entspricht 1.3 Äquivalenten) zugesetzt. Diese Mischung wurde beim Raumtemperatur für 1,5 Stunden zwecks Voraktivierung der pll gerührt.

Anschließend wurden 50 mg *trans*-Chalkon zugesetzt und die Reaktionsmischung für 10 min Reaktionszeit gerührt. Im Anschluß wurde die Reaktionsmischung mit 2 ml Ethylacetat verdünnt und anschließend langsam in eine gerührte, eiskalte wäßrige NaHSO₃ Lösung eingetragen (4 ml, 20%ig). Nach Zentrifugation wurde der Überstand über Natriumsulfat getrocknet und unter verringertem Druck eingeengt.

### Vergleichsbeispiele 3 und 4: 3-phasige Bedingungen mit PTC ohne pll-Voraktivierung

100 mg nicht voraktivierter pll, 50 mg *trans*-Chalkon sowie 8.5 mg (Bu₄N)⁺Br⁻(Vergleichsbeispiel 3) bzw. 11 mg Aliquat® 336 (Vergleichsbeispiel 4) wurden in einer Mischung aus 0.8 ml Toluol und 62 µl NaOH (eingesetzt als 5 molare wässrige Lösung, entspricht 1.3 Äquivalenten) suspendiert. Anschließend wurden 32µl H₂O₂ (eingesetzt als 30%ige wässrige Lösung, 1,3 Äquivalente) zugesetzt. Diese Mischung wurde bei Raumtemperatur unter Rühren umgesetzt. Nach 10 min Reaktionszeit wurde die Reaktionsmischung mit 2 ml Ethylacetat verdünnt und anschließend langsam in eine gerührte, eiskalte wäßrige NaHSO₃ Lösung eingetragen (4 ml, 20%ig). Nach Zentrifugation wurde der Überstand über Natriumsulfat getrocknet und unter verringertem Druck eingeengt.

Die Ergebnisse der Beispiele 1 und 2 sowie der Vergleichsbeispiele VB 3 und 4 sind in der nachfolgenden Tabelle 1 zusammengefasst.

**Tabelle 1:**

| Beispiel | PTC | Reaktionszeit [min] | Umsatz [%] | *ee* [%] |
|---|---|---|---|---|
| 1 | (Bu₄N)⁺Br⁻ | 10 | 97 | 95 |
| 2 | Aliquat® 336 | 10 | 74 | 94 |
| VB 3 | (Bu₄N)⁺Br⁻ | 10 | 97 | 94 |
| VB 4 | Aliquat® 336 | 10 | 67 | 89 |

### Beispielgruppe II:

### Beispiele 5 und 6 sowie Vergleichsbeispiele VB 7 - 8

### Epoxidierung von trans-Aminochalkon (3) zu (4)

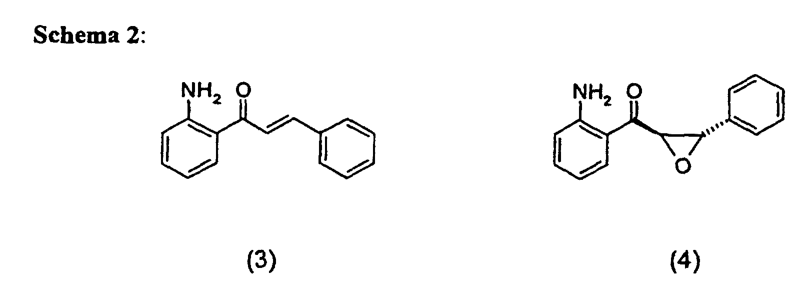

### Beispiele 5 und 6: 3-phasige Bedingungen mit PTC und mit pll-Voraktivierung

100 mg nicht voraktivierter pll sowie 8.5 mg (Bu₄N)⁺Br⁻ (bzw. 10.8 mg Aliquat®336) wurden in einer Mischung aus 0.8 ml Toluol und 200 µl NaOH (eingesetzt als 5 molare wässrige Lösung, entspricht 4.2 Äquivalenten) suspendiert. Anschließend wurden 125 µl H₂O₂ (eingesetzt als 30%ige wässrige Lösung, entspricht 5.0 Äquivalenten) zugesetzt. Diese Mischung wurde bei Raumtemperatur für 1,5 Stunden zwecks Voraktivierung der pll gerührt.

Anschließend wurden 54 mg *trans*-Aminochalkon zugesetzt und die Reaktionsmischung für 30 min Reaktionszeit gerührt. Im Anschluß wurde die Reaktionsmischung mit 2 ml Ethylacetat verdünnt und anschließend langsam in eine gerührte, eiskalte wäßrige NaHSO₃ Lösung eingetragen (4 ml, 20%ig). Nach Filtration wurde die organische Phase des Filtrats über Natriumsulfat getrocknet und unter verringertem Druck eingeengt.

### Vergleichsbeispiele 7 und 8: 3-phasige Bedingungen mit PTC ohne pll-Voraktivierung

100 mg nicht voraktivierter pll, 54 mg *trans*-Aminochalkon sowie 8.5 mg (Bu₄N)⁺Br⁻(bzw. 10.8 mg Aliquat 336) wurden in einer Mischung aus 0.8 ml Toluol und 200 µl NaOH (eingesetzt als 5 molare wässrige Lösung, entspricht 4.2 Äquivalenten) suspendiert. Anschließend wurden 125 µl H₂O₂ (eingesetzt als 30%ige wässrige Lösung, entspricht 5.0 Äquivalenten) zugesetzt. Diese Mischung wurde bei Raumtemperatur unter Rühren umgesetzt. Nach 30 min Reaktionszeit wurde die Reaktionsmischung mit 2 ml Ethylacetat verdünnt und anschließend langsam in eine gerührte, eiskalte wäßrige NaHSO₃ Lösung eingetragen (4 ml, 20%ig). Nach Filtration wurde die organische Phase des Filtrats über Natriumsulfat getrocknet und unter reduziertem Druck eingeengt.

Die Ergebnisse der Beispiele 5 und 6 sowie der Vergleichsbeispiele VB 7 und 8 sind in der nachfolgenden Tabelle 2 zusammengefasst.

**Tabelle 2:**

| Beispiel | PTC | Reaktionszeit [min] | Umsatz [%] | *ee* [%] |
|---|---|---|---|---|
| 5 | (Bu₄N)⁺Br⁻ | 30 | 100 | 92 |
| 6 | Aliquat® 336 | 30 | 100 | 91 |
| VB 7 | (Bu₄N)⁺Br⁻ | 30 | 89 | 87 |
| VB 8 | Aliquat® 336 | 30 | 74 | 55 |

### Beispielgruppe III:

### Beispiele 9 und 10 sowie Vergleichsbeispiele 11 und 12

### Epoxidierung von Phenyl-trans-styrylsulfon (5) zu (6)

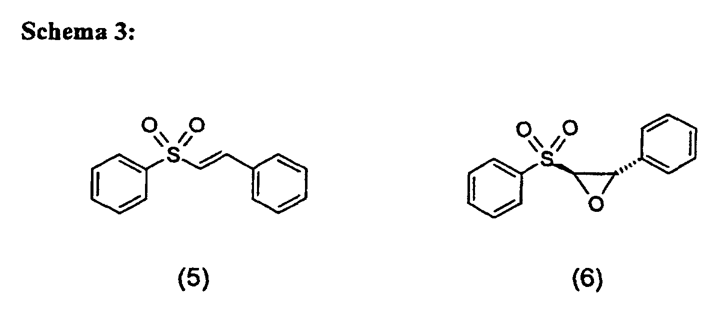

### Beispiele 9 und 10: 3-phasige Bedingungen mit PTC und mit pll-Voraktivierung

100 mg nicht voraktivierter pll sowie 8.5 mg (Bu₄N)⁺Br⁻ Beispiel 9 (bzw. 11 mg Aliquat 336)Beispiel 10 wurden in einer Mischung aus 0.8 ml Toluol und 200 µl NaOH (eingesetzt als 5 molare wässrige Lösung, entspricht 4,2 Äquivalenten) suspendiert. Anschließend wurden 120 µl H₂O₂ (eingesetzt als 30%ige wässrige Lösung, entspricht 5 Äquivalenten) zugesetzt. Diese Mischung wurde bei Raumtemperatur für 1,5 Stunden zwecks Voraktivierung der pll gerührt.

Anschließend wurden dann 59 mg Phenyl-*trans*-styrylsulfon zugesetzt und für weitere 2 Stunden gerührt. Die Reaktionsmischung wurde mit 2 ml Ethylacetat verdünnt und anschließend zentrifugiert. Der Überstand wurde dann langsam in 2 ml Wasser eingetragen. Nach der Phasentrennung wurde die organische Phase über Natriumsulfat getrocknet und unter verringertem Druck eingeengt.

### Vereleichsbeispiele 11 und 12: 3-phasige-Bedingungen mit PTC ohne pll-Voraktivierung

100 mg nicht voraktivierter pll, 59 mg Phenyl-*trans*-styrylsulfon sowie 8.5 mg (Bu₄N)⁺Br⁻ (VB 11) (bzw. 11 mg Aliquat®336) (VB 12)wurden in einer Mischung aus 0.8 ml Toluol und 200 µl NaOH (eingesetzt als 5 molare wässrige Lösung, entspricht 4,2 Äquivalenten) suspendiert. Anschließend wurden 125 µl H₂O₂ (eingesetzt als 30%ige wässrige Lösung, entspricht 5 Äquivalenten) zugesetzt. Diese Mischung wurde bei Raumtemperatur unter Rühren umgesetzt. Nach 2 h Reaktionszeit wurde die Reaktionsmischung mit 2 ml Ethylacetat verdünnt und anschließend zentrifugiert. Der Überstand wurde dann langsam in 2 ml Wasser eingetragen. Nach der Phasentrennung wurde die organische Phase über Natriumsulfat getrocknet und unter verringertem Druck eingeengt.

Die Ergebnisse der Beispiele 9 und 10 sowie der Vergleichsbeispiele VB 11und 12 sind in der nachfolgenden Tabelle 3 zusammengefasst.

**Tabelle 3:**

| Beispiel | PTC | Reaktionszeit [h] | Umsatz [%] | *ee* [%] |
|---|---|---|---|---|
| 9 | (Bu₄N)⁺Br⁻ | 2 | 82 | 68 |
| 10 | Aliquat® 336 | 2 | 95 | 65 |
| VB11 | (Bu₄N)⁺Br⁻ | 2 | 79 | 53 |
| VB 12 | Aliquat® 336 | 2 | 92 | 22 |

### Beispielgruppe IV:

### Beispiel 13 und Vergleichsbeispiel 14

### Epoxidierung von (E)-1,2-Dibenzoylethylen (7) zu (8)

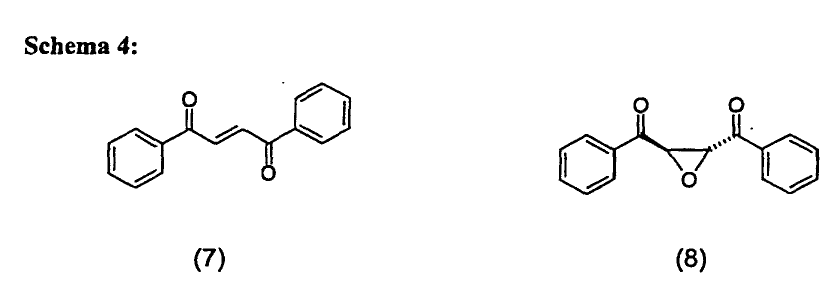

### Beispiel 13: 3-phasige Bedingungen mit PTC und mit pll-Voraktivierung

100 mg nicht voraktivierte pll (11 mol%), und 8.5 mg (Bu₄N)⁺Br⁻ (11 mol%) wurden in einer Mischung aus 0.8 ml Toluol und 63 µl NaOH (eingesetzt als 5 molare wässrige Lösung, entspricht 1.3 Äquivalenten) suspendiert. Anschließend wurden 32 µl H₂O₂ (eingesetzt als 30%ige wässrige Lösung, entspricht 1.3 Äquivalenten) zugesetzt. Diese Mischung wurde bei Raumtemperatur für 1,5 Stunden zwecks Voraktivierung der pll gerührt.

Anschließend wurden 57 mg (*E*)-1,2-Dibenzoylethylen zugesetzt und die Reaktionsmischung für 8 min Reaktionszeit gerührt. Die Reaktionsmischung wurde mit 2 ml Ethylacetat verdünnt und anschließend langsam in eine gerührte, eiskalte wäßrige NaHSO₃-Lösung eingetragen (4 ml, 20%ig). Nach Abfiltrieren des Polymers wurde die organische Phase des Filtrats über Natriumsulfat getrocknet und unter verringertem Druck eingeengt. Es wird ein Umsatz von 100% und ein Enantiomerenüberschuß von 92% *ee* erhalten (bestimmt durch Shift-¹H-NMR-Experiment mit Eu(tfc)₃ als Shift-Reagenz).

### Vergleichsbeispiel 14: 3-phasige-Bedingungen mit PTC ohne pll-Voraktivierung

100 mg nicht voraktivierte pll (11 mol%), 57 mg (*E*)-1,2-Dibenzoylethylen sowie 8.5 mg (Bu₄N)⁺Br⁻ (11 mol%) wurden in einer Mischung aus 0.8 ml Toluol und 63 µl NaOH (eingesetzt als 5 molare wässrige Lösung, entspricht 1.3 Äquivalenten) suspendiert. Anschließend wurden 32 µl H₂O₂ (eingesetzt als 30%ige wässrige Lösung, entspricht 1.3 Äquivalenten) zugesetzt. Diese Mischung wurde beim Raumtemperatur unter Rühren umgesetzt. Nach 8 min Reaktionszeit wurde die Reaktionsmischung mit 2 ml Ethylacetat verdünnt und anschließend langsam in eine gerührte, eiskalte wäßrige NaHSO₃-Lösung eingetragen (4 ml, 20%ig). Nach Abfiltrieren des Polymers wurde die organische Phase des Filtrats über Natriumsulfat getrocknet und unter verringertem Druck eingeengt. Es wird ein Umsatz von 100% und ein Enantiomerenüberschuß von 69% *ee* erhalten (bestimmt durch Shift-¹H-NMR-Experiment mit Eu(tfc)₃ als Shift-Reagenz).

### Beispielgruppe V:

### Beispiel 15 und Vergleichsbeispiel 16

### Epoxidierung von (E)-1,2-Dibenzoylethylen (7) zu (8) gemäß Schema 4

### Beispiel 15: 3-phasige Bedingungen mit PTC und mit pIl-Voraktivierung

100 mg nicht voraktivierte pll (11 mol%), und 14.5 mg (Oct₄N)⁺Br⁻ (11 mol%) wurden in einer Mischung aus 0.8 ml Toluol und 63 µl NaOH (eingesetzt als 5 molare wässrige Lösung, entspricht 1.3 Äquivalenten) suspendiert. Anschließend wurden 32 µl H₂O₂ (eingesetzt als 30%ige wässrige Lösung, entspricht 1.3 Äquivalenten) zugesetzt. Diese Mischung wurde bei Raumtemperatur für 1,5 Stunden zwecks Voraktivierung der pll gerührt.

Anschließend wurden 57 mg (*E*)-1,2-Dibenzoylethylen zugesetzt und die Reaktionsmischung für 15 min Reaktionszeit gerührt. Die Reaktionsmischung wurde mit 2 ml Ethylacetat verdünnt und anschließend langsam in eine gerührte, eiskalte wäßrige NaHSO₃-Lösung eingetragen (4 ml, 20%ig). Nach Abfiltrieren des Polymers wurde die organische Phase des Filtrats über Natriumsulfat getrocknet und unter verringertem Druck eingeengt. Es wird ein Umsatz von 100% und ein Enantiomerenüberschuß von 95% *ee* erhalten (bestimmt durch Shift-¹H-NMR-Experiment mit Eu(tfc)₃ als Shift-Reagenz).

### Vergleichsbeispiel 16: 3-phasige-Bedingungen mit PTC ohne pll-Voraktivierung

100 mg nicht voraktivierte pll (11 mol%), 57 mg (*E*)-1,2-Dibenzoylethylen sowie 14.5 mg (Oct₄N)⁺Br⁻ (11 mol%) wurden in einer Mischung aus 0.8 ml Toluol und 63 µl NaOH (eingesetzt als 5 molare wässrige Lösung, entspricht 1.3 Äquivalenten) suspendiert. Anschließend wurden 32 µl H₂O₂ (eingesetzt als 30%ige wässrige Lösung, entspricht 1.3 Äquivalenten) zugesetzt. Diese Mischung wurde beim Raumtemperatur unter Rühren umgesetzt. Nach 15 min Reaktionszeit wurde die Reaktionsmischung mit 2 ml Ethylacetat verdünnt und anschließend langsam in eine gerührte, eiskalte wäßrige NaHSO₃-Lösung eingetragen (4 ml, 20%ig). Nach Abfiltrieren des Polymers wurde die organische Phase des Filtrats über Natriumsulfat getrocknet und unter verringertem Druck eingeengt. Es wird ein Umsatz von 100% und ein Enantiomerenüberschuß von 82% *ee* erhalten (bestimmt durch Shift-¹H-NMR-Experiment mit Eu(tfc)₃ als Shift-Reagenz).

### Beispiel 17: Epoxidierung von Benzylidenaceton (11) zu (12) (dreiphasige Bedingungen mit PTC und mit Voraktivierung)

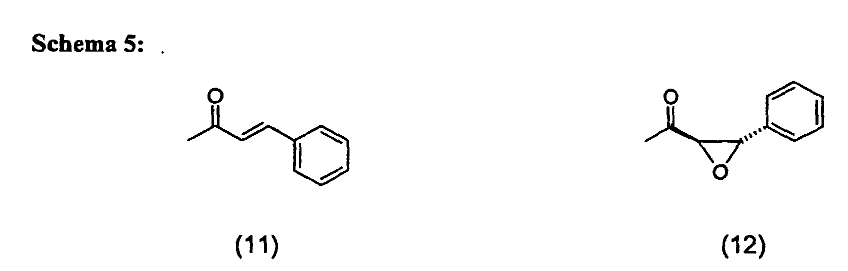

200 mg nicht voraktivierte pll (11 mol%), und 17 mg (Bu₄N)⁺Br⁻ (11 mol%) wurden in einer Mischung aus 1.6 ml Toluol und 0.4 ml NaOH (eingesetzt als 5 molare wässrige Lösung, entspricht 4.2 Äquivalenten) suspendiert. Anschließend wurden 0.25 ml H₂O₂ (eingesetzt als 30%ige wässrige Lösung, entspricht 5.0 Äquivalenten) zugesetzt. Diese Mischung wurde bei Raumtemperatur für 1,5 Stunden zwecks Voraktivierung der pll gerührt.

Anschließend wurden 70 mg Benzylidenaceton zugesetzt und die Reaktionsmischung für 1 Stunde Reaktionszeit gerührt. Die Reaktionsmischung wurde mit 2 ml Ethylacetat verdünnt und und anschließend zentrifugiert. Der Überstand wurde dann langsam in 2 ml Wasser eingetragen. Nach der Phasentrennung wurde die organische Phase über Natriumsulfat getrocknet und unter verringertem Druck eingeengt. Es wird bei einem Umsatz von 83% eine Ausbeute von 64% und ein Enantiomerenüberschuß von 77% *ee* erhalten (bestimmt durch Shift-¹H-NMR-Experiment).

## Patentansprüche

1. Verfahren zur Epoxidierung von α,β-ungesättigten Enonen oder α,β-ungesättigten Sulfonen in Gegenwart
(1) einer wasserlöslichen Base,
(2) eines Oxidationsmittels,
(3) einer diastereomeren- und enantiomerenangereicherten Homo-Polyaminosäure als Katalysator,
(4) Wasser und
(5) eines mit Wasser nicht oder nur begrenzt mischbaren Lösungsmittels,
**dadurch gekennzeichnet, dass** die Epoxidierung
(6) in Gegenwart eines Phasentransferkatalysators durchgeführt wird und die Homo-Polyaminosäure vor der Epoxidierung in Gegenwart des Phasentransferkatalysators einer Voraktivierung unterworfen wird.

2. Verfahren nach Anspruch 1, **dadurch gekennzeichnet, dass** als Phasentransferkatalysator quartäre Ammoniumsalze, quartäre Phosphoniumsalze, Onium-Verbindungen oder Pyridiniumsalze eingesetzt werden.

3. Verfahren nach Anspruch 2, **dadurch gekennzeichnet, dass** als Phasentransferkatalysatoren quartäre Ammonium- oder Phosphoniumsalze der allgemeinen Formel (I) eingesetzt werden,
(R¹R²R³R⁴A)⁺X⁻ (I)
worin
A für N oder P steht,
X' für ein anorganisches oder organisches Anion steht,
R¹, R² , R³ und R⁴ gleich oder verschieden sind und für Alkyl-, Aryl-, Aralkyl-, Cycloalkyl- oder Heteroaryl-Reste stehen, die durch einen oder mehrere, gleiche oder verschiedene Halogenreste substituiert sein können, oder aber jeweils zwei Reste unter Einbindung von A einen C₄-C₆-Cycloalkyl-Ring bilden können.

4. Verfahren nach Anspruch 3, **dadurch gekennzeichnet, dass** in der allgemeinen Formel (I) X für F⁻, Cl⁻, Br⁻, I⁻, OH⁻, NO₃⁻, HSO₄⁻, SO₄⁻, CH₃COO⁻, CF₃COO⁻, C₂H₅COO⁻, C₃H₇COO⁻, CF₃SO₃⁻ oder C₄F₉SO₃⁻ steht.

5. Verfahren nach einem oder mehreren der Ansprüche 2-4, **dadurch gekennzeichnet, dass** solche Phasentransferkatalysatoren der allgemeinen Formel (I) eingesetzt werden, bei denen R¹, R² , R³ und R⁴ gleich oder verschieden sind und für C₁-C₁₈-Alkyl, C₆-C₁₈-Aryl, C₇-C₁₉-Aralkyl, C₅-C₇-Cycloalkyl oder C₃-C₁₈-Heteroaryl stehen.

6. Verfahren nach Anspruch 3, **dadurch gekennzeichnet, dass** als Phasentransferkatalysator ((C₄H₉)₄N)⁺Hal⁻, bevorzugt ((C₄H₉)₄N)⁺Br⁻, ((C₄H₉)₄P)⁺Hal⁻, bevorzugt ((C₄H₉)₄P)⁺Br⁻, ((C₄H₉)₄N)⁺HSO₄⁻, ((C₈H₁₇)₄N)⁺Br⁻, [(CH₃)(C₈H₁₇)₃N] ⁺Cl⁻ oder [(CH₃)(C₄H₉)₃ N]⁺Cl⁻ eingesetzt werden.

7. Verfahren nach einem oder mehreren der Ansprüche 1-6, **dadurch gekennzeichnet, dass** der Phasentransferkatalysator in einer Menge im Bereich von 0.1 - 20 mol%, bevorzugt im Bereich von 0,5-15 mol%, besonders bevorzugt im Bereich von 0,5-11 mol%, jeweils bezogen auf das eingesetzte α,β-ungesättigte Enon oder α,β-ungesättigte Sulfon, eingesetzt wird.

8. Verfahren nach einem oder mehreren der Ansprüche 1-7, **dadurch gekennzeichnet, dass** als α,β-ungesättigte Enone oder α,β-ungesättigte Sulfone Verbindungen der allgemeinen Formel (II) 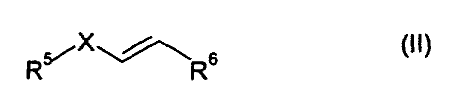 eingesetzt werden, worin
X für (C=O) oder (SO₂) steht und
R⁵ und R⁶ gleich oder verschieden sind und (C₁-C₁₈)-Alkyl, (C₂-C₁₈)-Alkenyl, (C₂-C₁₈)-Alkinyl, (C₃-C₈)-Cycloalkyl, (C₆-C₁₈)-Aryl, (C₇-C₁₉)-Aralkyl, (C₁-C₁₈)-Heteroaryl oder (C₂-C₁₉)-Heteroarakyl bedeuten,
wobei die für R⁵ und R⁶ genannten Reste einfach oder mehrfach mit gleichen oder verschiedenen Resten Rest R⁷, Halogen, NO₂, NR⁷R⁸, PO₀₋₃R⁷R⁸, SO₀₋₃R⁷, OR⁷, CO₂R⁷, CONHR⁷ oder COR⁷ substituiert sein können und gegebenenfalls eine oder mehrere CH₂-Gruppen in den Resten R⁵ und R⁶ durch O, SO₀₋₂, Nur oder PO₀₋₂R⁷ substituiert sein können,
wobei R⁷ und R⁸ gleich oder verschieden sind und H, (C₁-C₁₈)-Alkyl, (C₂-C₁₈)-Alkenyl, (C₂-C₁₈)-Alkinyl, (C₃-C₈)-Cycloalkyl, (C₆-C₁₈)-Aryl, (C₁-C₁₈)-Heteroaryl, (C₁-C₈)-Alkyl-(C₆-C₈)-Aryl, (C₁-C₈)-Alkyl-(C₁-C₁₈)-Heteroaryl, (C₁-C₈)-Alkyl-(C₃-C₈)-Cycloalkyl bedeuten und diese Reste R⁷ und R⁸ einfach oder mehrfach mit gleichen oder verschiedenen Halogenresten substituiert sein können.

9. Verfahren nach einem oder mehreren der Ansprüche 1-8, **dadurch gekennzeichnet, dass** als α,β-ungesättigte Enone oder α,β-ungesättigte Sulfone Verbindungen der allgemeinen Formel (II) eingesetzt werden, in denen R⁵ und R⁶ gleich oder verschieden sind und (C₁-C₁₂)-Alkyl, (C₂-C₁₂)-Alkenyl, (C₂-C₁₂)-Alkinyl, (C₅-C₈)-Cycloalkyl, (C₆-C₁₂)-Aryl oder (C₁-C₁₂)-Heteroaryl bedeuten, wobei die zuvor genannten Reste einfach oder mehrfach mit gleichen oder verschiedenen Resten R⁷, Halogen, NO₂, NR⁷R⁸, PO₀₋₃R⁷R⁸ oder OR⁷ substituiert sein können und R⁷ und R⁸ die für die allgemeine Formel (II) angegebenen Bedeutungen besitzen.

10. Verfahren nach einem oder mehreren der Ansprüche 1-9, **dadurch gekennzeichnet, dass** als α,β-ungesättigte Enone oder α,β-ungesättigte Sulfone der allgemeinen Formel (II) eingesetzt werden, in denen R⁵ und R⁶ gleich oder verschieden sind und (C₁-C₁₂)-Alkyl, (C₂-C₁₂)-Alkenyl, (C₂-C₁₂)-Alkinyl, (C₅-C₈)-Cycloalkyl, (C₆-C₁₂)-Aryl oder (C₁-C₁₂)-Heteroaryl bedeuten, wobei die zuvor genannten Reste einfach oder mehrfach mit gleichen oder verschiedenen Resten R⁷, Halogen, NO₂, NR⁷R⁸, PO₀₋₃R⁷R⁸ oder OR⁷ substituiert sein können und R⁷ und R⁸ die für die allgemeine Formel (II) angegebene Bedeutungen besitzen, mit der Maßgabe, dass mindestens einer der Reste R⁵ oder R⁶ einen (C₂-C₁₂)-Alkenyl-, (C₂-C₁₂)-Alkinyl-, (C₆-C₁₂)-Aryl- oder (C₁-C₁₂)-Heteroarylrest darstellt.

11. Verfahren nach einem oder mehreren der Ansprüche 1-10, **dadurch gekennzeichnet, dass** als α,β-ungesättigte Enone oder α,β-ungesättigte Sulfone Verbindungen der allgemeinen Formel (III) eingesetzt werden, 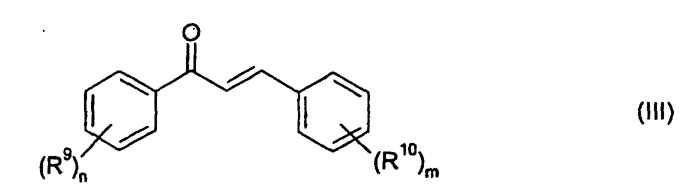 wobei
n und m gleich oder verschieden sind und für die Zahlen 0, 1, 2 oder 3 stehen und
R⁹ und R¹⁰ gleich oder verschieden sind und NR⁷R⁸, NO₂, OR⁷, (C₁-C₁₂)-Alkyl, (C₂-C₁₂)-Alkenyl, (C₂-C₁₂)-Alkinyl, (C₅-C₈)-Cyclo-alkyl, (C₆-C₁₂)-Aryl oder (C₁-C₁₂)-Heteroaryl bedeuten, wobei diese Reste R⁹ und R¹⁰ einfach oder mehrfach mit gleichen oder verschiedenen Halogenresten substituiert sein können und R ⁷ und R⁸ die für Formel (II) genannten Bedeutungen besitzen.

12. Verfahren nach einem oder mehreren der Ansprüche 1-11, **dadurch gekennzeichnet, dass** als diastereomeren- und enantiomerenangereicherten Homo-Polyaminosäuren solche aus der Gruppe Polyneopentylglycin, Polyleucin, Polyisoleucin, Polyvalin, Polyalanin und Polyphenylalanin verwendet werden.

13. Verfahren nach einem oder mehreren der Ansprüche 1-12, **dadurch gekennzeichnet, dass** die Kettenlänge der Polyaminosäuren im Bereich von 5 bis 100, vorzugsweise im Bereich von 7 bis 50 und insbesondere im Bereich von 10 bis 40 Aminosäure-Wiederholungseinheiten liegt.

14. Verfahren nach einem oder mehreren der Ansprüche 1-13, **dadurch gekennzeichnet, dass** die Homo-Polyaminosäure zwecks Voraktivierung zusammen mit dem Phasentransferkatalysator in Gegenwart der Base und des Oxidationsmittels in einem organischen Lösungsmittel suspendiert und für einen Zeit von 15 Minuten bis 2 Stunden gerührt wird, und anschließend entweder zwischenisoliert wird oder aber bevorzugt das Reaktionssystem nach Zugabe des α,β-ungesättigten Enons oder α,β-ungesättigten Sulfons unmittelbar zur Epoxidierung verwendet wird.

15. Verfahren nach einem oder mehreren der Ansprüche 1-14, **dadurch gekennzeichnet, dass** die Homo-Polyaminosäure im Bereich von 0.1 - 40 mol%, bevorzugt im Bereich von 0,5-20 mol%, besonders bevorzugt im Bereich von 1-15 mol%, jeweils bezogen auf das eingesetzte α,β-ungesättigte Enon oder α,β-ungesättigte Sulfon, eingesetzt wird.

16. Verfahren nach einem oder mehreren der Ansprüche 1-15, **dadurch gekennzeichnet, dass** als Oxidationsmittel Peroxide, Persäuren oder anorganische Oxidationsmittel wie Natriumhypochlorit oder Natriumpercarbonat eingesetzt werden.

17. Verfahren nach einem oder mehreren der Ansprüche 1-16, **dadurch gekennzeichnet, dass** als Oxidationsmittel eine wässrige H₂O₂-Lösung eingesetzt wird.

18. Verfahren nach einem oder mehreren der Ansprüche 1-17, **dadurch gekennzeichnet, dass** das Oxidationsmittel in einer Menge von 1 - 40 Äquivalenten, bevorzugt von 1 - 10 Äquivalenten, besonders bevorzugt von 1-3 Äquivalenten und insbesondere 1,1 - 2,5 Äquivalenten eingesetzt wird.

19. Verfahren nach einem oder mehreren der Ansprüche 1-18, **dadurch gekennzeichnet, dass** als wasserlöslichen Base ein Alkalimetallhydroxid, bevorzugt NaOH, KOH oder LiOH eingesetzt wird.

20. Verfahren nach einem oder mehreren der Ansprüche 1-19, **dadurch gekennzeichnet, dass** die Base in einer Menge von 0,1-10 Äquivalenten, bevorzugt von 0,5-5 Äquivalenten und besonders bevorzugt von 0,8-2 Äquivalenten eingesetzt wird.

21. Verfahren nach einem oder mehreren der Ansprüche 1-20, **dadurch gekennzeichnet, dass** als organisches Lösungsmittel unsubstituierte oder substituierte aromatische Kohlenwasserstoffe, bevorzugt Toluol oder Xylol, aliphatische Kohlenwasserstoffe, bevorzugt Hexan, Halogenalkane, bevorzugt Chloroform oder Methylenchlorid, oder Ether, bevorzugt *tert.-Butyl* methylether und Diethylether eingesetzt werden.

22. Verfahren nach einem oder mehreren der Ansprüche 1-21, **dadurch gekennzeichnet, dass** die Reaktiontemperatur im Bereich von -10 bis +50 °C, bevorzugt im Bereich von 0 bis +40 °C und insbesondere bei +10 bis +30 °C liegt.

## Claims

1. Process for the epoxidation of α,β-unsaturated enones or α,β-unsaturated sulphones in the presence of
(1) a water-soluble base,
(2) an oxidant,
(3) a diastereomer- and enantiomer-enriched homo-polyamino acid as catalyst,
(4) water and
(5) a solvent which is immiscible or has only limited miscibility with water,
**characterized in that** the epoxidation is carried out
(6) in the presence of a phase-transfer catalyst and the homo-polyamino acid is subjected to a preactivation in the presence of the phase-transfer catalyst before the epoxidation.

2. Process according to Claim 1, **characterized in that** quaternary ammonium salts, quaternary phosphonium salts, onium compounds or pyridinium salts are employed as phase-transfer catalyst.

3. Process according to Claim 2, **characterized in that** the phase-transfer catalysts employed are quaternary ammonium or phosphonium salts of the general formula (I)
(R¹R²R³R⁴A)⁺X⁻ (I)
where
A is N or P,
X⁻ is an inorganic or organic anion,
R¹, R², R³ and R⁴ are identical or different and are alkyl, aryl, aralkyl, cycloalkyl or heteroaryl radicals which may be substituted by one or more identical or different halogen radicals, or else two radicals in each case may form a C₄-C₆-cycloalkyl ring including A.

4. Process according to Claim 3, **characterized in that** X in the general formula (I) is F⁻, Cl⁻, Br⁻, I⁻, OH⁻, NO₃⁻, HSO₄⁻, SO₄⁻, CH₃COO⁻, CF₃COO⁻, C₂H₅COO⁻, C₃H₇COO⁻, CF₃SO₃⁻ or C₄F₉SO₃⁻.

5. Process according to one or more of Claims 2-4, **characterized in that** phase-transfer catalysts of the general formula (1) in which R¹, R², R³ and R⁴ are identical or different and are C₁-C₁₈-alkyl, C₆-C₁₈-aryl, C₇-C₁₉-aralkyl, C₅-C₇-cycloalkyl or C₃-C₁₈-heteroaryl are employed.

6. Process according to Claim 3, **characterized in that** ((C₄H₉)₄N)⁺Hal⁻, preferably ((C₄H₉)₄N)⁺Br⁻, ((C₄H₉)₄P)⁺Hal⁻, preferably ((C₄H₉)₄P)⁺Br⁻, ((C₄H₉)₄N)⁺HSO₄⁻, ((C₈H₁₇)₄N)⁺Br⁻, [(CH₃)(C₈H₁₇)₃N]⁺Cl⁻ or [(CH₃)(C₄H₉)₃N]⁺Cl⁻ are employed as phase-transfer catalyst.

7. Process according to one or more of Claims 1-6, **characterized in that** the phase-transfer catalyst is employed in an amount in the range 0.1-20 mol%, preferably in the range 0.5-15 mol%, particularly preferably in the range 0.5-11 mol%, in each case based on the α,β-unsaturated enone or α,β-unsaturated sulphone employed.

8. Process according to one or more of Claims 1-7, **characterized in that** the compounds employed as α,β-unsaturated enones or α,β-unsaturated sulphones have the general formula (II) 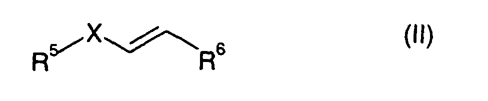 in which
X is (C=O) or (SO₂), and
R⁵ and R⁶ are identical or different and are (C₁-C₁₈)-alkyl, (C₂-C₁₈)-alkenyl, (C₂-C₁₈)-alkynyl, (C₃-C₈)-cycloalkyl, (C₆-C₁₈)-aryl, (C₇-C₁₉)-aralkyl, (C₁-C₁₈)-heteroaryl or (C₂-C₁₉)-heteroaralkyl,
where the radicals mentioned for R⁵ and R⁶ may be substituted once or more than once by identical or different radicals R⁷, halogen, NO₂, NR⁷R⁸, PO₀₋₃R⁷R⁸, SO₀₋₃R⁷, OR⁷, CO₂R⁷, CONHR⁷ or COR⁷, and optionally one or more CH₂ groups in the radicals R⁵ and R⁶ are replaced by O, SO₀₋₂, NR⁷ or PO₀₋₂R⁷,
where R⁷ and R⁸ are identical or different and are H, (C₁- C₁₈)-alkyl, (C₂-C₁₈)-alkenyl, (C₂-C₁₈)-alkynyl, (C₃-C₈)-cycloalkyl, (C₆-C₁₈)-aryl, (C₁-C₁₈)-heteroaryl, (C₁-C₈)-alkyl-(C₆-C₈)-aryl, (C₁-C₈)-alkyl-(C₁-C₁₈)-heteroaryl, (C₁-C₈)-alkyl-(C₃-C₈)-cycloalkyl, and these radicals R⁷ and R⁸ may be substituted once or more than once by identical or different halogen radicals.

9. Process according to one or more of Claims 1-8, **characterized in that** the compounds employed as α,β-unsaturated enones or α,β-unsaturated sulphones have the general formula (II) in which R⁵ and R⁶ are identical or different and are (C₁-C₁₂)-alkyl, (C₂-C₁₂)-alkenyl, (C₂-C₁₂)-alkynyl, (C₅-C₈)-cycloalkyl, (C₆-C₁₂)-aryl or (C₁-C₁₂)-heteroaryl, where the aforementioned radicals may be substituted once or more than once by identical or different radicals R⁷, halogen, NO₂, NR⁷R⁸, PO₀₋₃R⁷R⁸ or OR⁷, and R⁷ and R⁸ have the meanings indicated above for the general formula (II).

10. Process according to one or more of Claims 1-9, **characterized in that** it utilizes α,β-unsaturated enones or α,β-unsaturated sulphones of the general formula (II) in which R⁵ and R⁶ are identical or different and are (C₁-C₁₂)-alkyl, (C₂-C₁₂)-alkenyl, (C₂-C₁₂)-alkynyl, (C₅-C₈)-cycloalkyl, (C₆-C₁₂)-aryl or (C₁-C₁₂)-heteroaryl, where the aforementioned radicals may be substituted once or more than once by identical or different radicals R⁷, halogen, NO₂, NR⁷R⁸, PO₀₋₃R⁷R⁸ or OR⁷, and R⁷ and R⁸ have the meanings indicated above for the general formula (II), with the proviso that at least one of the radicals R⁵ or R⁶ is a (C₂-C₁₂)-alkenyl, (C₂-C₁₂)-alkynyl, (C₆-C₁₂)-aryl- or (C₁-C₁₂)-heteroaryl radical.

11. Process according to one or more of Claims 1-10, **characterized in that** the α,β-unsaturated enones or α,β-unsaturated sulphones employed are compounds of the general formula (III) 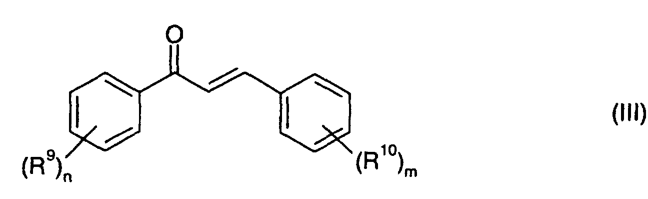 where
n and m are identical or different and are the numbers 0, 1, 2 or 3,
and
R⁹ and R¹⁰ are identical or different and are NR⁷R⁸, NO₂, OR⁷, (C₁-C₁₂)-alkyl, (C₂-C₁₂)-alkenyl, (C₂-C₁₂)-alkynyl, (C₅-C₈)-cycloalkyl, (C₆-C₁₂)-aryl or (C₁-C₁₂)-heteroaryl where these radicals R⁹ and R¹⁰ may be substituted once or more than once by identical or different halogen radicals, and R ⁷ and R⁸ have the meanings mentioned for formula (II).

12. Process according to one or more of Claims 1-11, **characterized in that** the diastereomer- and enantiomer-enriched homo-polyamino acids used are those from the group of polyneopentylglycine, polyleucine, polyisoleucine, polyvaline, polyalanine and polyphenylalanine.

13. Process according to one or more of Claims 1-12, **characterized in that** the chain length of the polyamino acids is in the range from 5 to 100, preferably in the range from 7 to 50, and in particular in the range from 10 to 40, amino acid repeating units.

14. Process according to one or more of Claims 1-13, **characterized in that** the homo-polyamino acids are for preactivation suspended together with the phase-transfer catalyst in the presence of the base and of the oxidant in an organic solvent and stirred for a time of from 15 minutes to 2 hours, and either then undergoes intermediate isolation or else, preferably, the reaction system is used directly for the epoxidation after addition of the α,β-unsaturated enone or α,β-unsaturated sulphone.

15. Process according to one or more of Claims 1-14, **characterized in that** the homo-polyamino acids are employed in the range 0.1-40 mol%, preferably in the range 0.5-20 mol%, particularly preferably in the range 1-15 mol%, in each case based on the α,β-unsaturated enone or α,β-unsaturated sulphone employed.

16. Process according to one or more of Claims 1-15, **characterized in that** peroxides, peracids or inorganic oxidants such as sodium hypochlorite or sodium percarbonate are employed as oxidants.

17. Process according to one or more of Claims 1-16, **characterized in that** an aqueous H₂O₂ solution is employed as oxidant.

18. Process according to one or more of Claims 1-17, **characterized in that** the oxidant is employed in an amount of 1-40 equivalents, preferably of 1-10 equivalents, particularly preferably of 1-3 equivalents and in particular 1.1-2.5 equivalents.

19. Process according to one or more of Claims 1-18, **characterized in that** an alkali metal hydroxide, preferably NaOH, KOH or LiOH, is employed as water-soluble base.

20. Process according to one or more of Claims 1-19, **characterized in that** the base is employed in an amount of 0.1-10 equivalents, preferably of 0.5-5 equivalents and particularly preferably of 0.8-2 equivalents.

21. Process according to one or more of Claims 1-20, **characterized in that** unsubstituted or substituted aromatic hydrocarbons, preferably toluene or xylene, aliphatic hydrocarbons, preferably hexane, haloalkanes, preferably chloroform or methylene chloride, or ethers, preferably *tert-*butyl methyl ether and diethyl ether, are employed as organic solvent.

22. Process according to one or more of Claims 1-21, **characterized in that** the reaction temperature is in the range from -10 to +50°C, preferably in the range from 0 to +40°C and in particular at +10 to +30°C.

## Revendications

1. Procédé pour l'époxydation d'énones α,β-insaturées ou de sulfones α,β-insaturées en présence de
(1) une base soluble dans l'eau,
(2) un agent oxydant,
(3) un homo-polyaminoacide enrichi en diastéréoisomères et énantiomères, qui sert de catalyseur,
(4) de l'eau, et
(5) un solvant non miscible à l'eau ou à miscibilité limitée avec l'eau,
**caractérisé en ce que** l'époxydation est réalisée
(6) en présence d'un catalyseur à transfert de phase et **en ce que** l'homo-polyaminoacide est soumis à une activation préalable avant l'époxydation en présence du catalyseur à transfert de phase.

2. Procédé selon la revendication 1, **caractérisé en ce que** le catalyseur à transfert de phase consiste en un sel d'ammonium quaternaire, un sel de phosphonium quaternaire, un composé en onium ou un sel de pyridinium.

3. Procédé selon la revendication 2, **caractérisé en ce que** l'on utilise en tant que catalyseurs à transfert de phase des sels d'ammonium ou de phosphonium quaternaires de formule générale (I)
(R¹R²R³R⁴A)⁺X⁻ (I)
dans laquelle
A représente N ou P,
X⁻ représente un anion inorqanique ou orqanique,
R¹, R², R³ et R⁴, ayant des significations identiques ou différentes, représentent chacun un groupe alkyle, aryle, aralkyle, cycloalkyle ou hétéroaryle, qui peut porter un ou plusieurs substituants halogénés, identiques ou différents, ou bien deux de ces groupes peuvent former avec A un noyau cycloalkyle en C₄-C₆.

4. Procédé selon la revendication 3, **caractérisé en ce que**, dans la formule générale (I), X représente F⁻, CI⁻, Br⁻, I⁻, OH⁻, HSO₄⁻, SO₄⁻, NO₃⁻, CH₃COO⁻, CF₃COO⁻, C₂H₅COO⁻, C₃H₇COO⁻, CF₃SO₃⁻ ou C₄F₉SO₃⁻.

5. Procédé selon une plusieurs des revendications 2 à 4, **caractérisé en ce que** l'on utilise des catalyseurs à transfert de phase de formule générale (I) dans laquelle R¹, R², R³ et R⁴ ayant des significations identiques ou différentes, représentent chacun un groupe alkyle en C₁-C₁₈, aryle en C₆-C₁₈, aralkyle en C₇-C₁₉, cycloalkyle en C₅-C₇ ou hétéroaryle en C₃-C₁₈.

6. Procédé selon la revendication 3, **caractérisé en ce que** l'on utilise en tant que catalyseur à transfert de phase ((C₄H₉)₄N)⁺Br⁻, de préférence ((C₄H₉)₄P)⁺Hal⁻, plus spécialement ((C₄H₉)₄P)⁺Br⁻, ((C₄H₉)₄N)⁺HSO₄⁻, ((C₈H₁₇)₄N)⁺Br⁻, [(CH₃)(C₈H₁₇)₃N⁺]Cl⁻ et [(CH₃)(C₄H₉)₃N⁺]Cl⁻.

7. Procédé selon une ou plusieurs des revendications 1 à 6, **caractérisé en ce que** le catalyseur à transfert de phase est mis en oeuvre en quantité de 0,1 à 20 mol%, de préférence de 0,5 à 15 mol%, plus spécialement de 0,5 à 11 mol%, dans tous les cas par rapport à l'énone α,β-insaturée ou à la sulfone α,β-insaturée mise en oeuvre.

8. Procédé selon une ou plusieurs des revendications 1 à 7, **caractérisé en ce que** l'on met en oeuvre une énone α,β-insaturée ou une sulfone α,β-insaturée répondant à la formule générale (II) 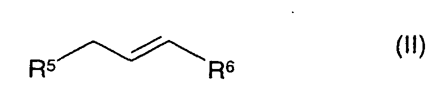 dans laquelle
X représente (C=O) ou (SO₂) et
R⁵ et R⁶, ayant des significations identiques ou différentes représentent chacun un groupe alkyle en C₁-C₁₈, alcényle en C₂-C₁₈, alcynyle en C₂-C₁₈, cycloalkyle en C₃-C₈, aryle en C₆-C₁₈, aralkyle en C₇-C₁₉, hétéroaryle en C₁-C₁₈ ou hétéroaralkyle en C₂-C₁₉,
chacun de ces groupes pouvant porter un ou plusieurs substituants R⁷, halogéno, NO₂, NR⁷R⁸, PO₀₋₃R⁷R⁸, SO₀₋₃R⁷, OR⁷, CO₂R⁷, CONHR⁷ ou COR⁷, et le cas échéant un ou plusieurs chaînons CH₂ des groupes R⁵ et R⁶ peuvent être remplacés par O, SO₀₋₂, NR⁷ ou PO₀₋₂R⁷,
R⁷ et R⁸, ayant des significations identiques ou différentes, représentent chacun H, un groupe alkyle en C₁-C₁₈, alcényle en C₂-C₁₈, alcynyle en C₂-C₁₈, cycloalkyle en C₃-C₈, aryle en C₆-C₁₈, hétéroaryle en C₁-C₁₈, (alkyle en C₁-C₈)-aryle en C₆-C₈, (alkyle en C₁-C₈)-hétéroaryle en C₁-C₁₉, (alkyle en C₁-C₈)-cycloalkyle en C₃-C₈, et ces groupes peuvent porter un ou plusieurs substituants halogénés identiques ou différents.

9. Procédé selon l'une ou plusieurs des revendications 1 à 8, **caractérisé en ce que** l'on met en oeuvre des énones α,β-insaturées ou des sulfones α,β-insaturées consistant en composés de formule générale (II) dans laquelle R⁵ et R⁶ ayant des significations identiques ou différentes représentent chacun un groupe alkyle en C₁-C₁₂, alcényle en C₂-C₁₂, alcynyle en C₂-C₁₂, cycloalkyle en C₅-C₈, aryle en C₆-C₁₂ ou hétéroaryle en C₁-C₁₂, chacun de ces groupes pouvant porter un ou plusieurs substituants identiques ou différents R⁷, halogéno, NO₂, NR⁷R⁸, PO₀₋₃R⁷R⁸ ou OR⁷, R⁷ et R⁸ ayant les significations indiquées en référence à la formule générale (II).

10. Procédé selon une ou plusieurs des revendications 1 à 9, **caractérisé en ce que** l'on met en oeuvre des énones α,β-insaturées ou des sulfones α,β-insaturées de formule générale (II) dans laquelle R⁵ et R⁶ ayant des significations identiques ou différentes représentent chacun un groupe alkyle en C₁-C₁₂, alcényle en C₂-C₁₂, alcynyle en C₂-C₁₂, cycloalkyle en C₅-C₈, aryle en C₆-C₁₂ ou hétéroaryle en C₁-C₁₂, chacun de ces groupes pouvant porter un ou plusieurs substituants identiques ou différents R⁷, halogéno, NO₂, NR⁷R⁸, PO₀₋₃R⁷R⁸ ou OR⁷, et R⁷ et R⁸ ayant les significations indiquées en référence à la formule générale (II), sous réserve qu'au moins un des symboles R⁵ ou R⁶ représente un groupe alcényle en C₂-C₁₂, alcynyle en C₂-C₁₂, aryle en C₆-C₁₂ ou hétéroaryle en C₁-C₁₂.

11. Procédé selon une ou plusieurs des revendications 1 à 10, **caractérisé en ce que** l'on met en oeuvre des énones α,β-insaturées ou des sulfones α,β-insaturées consistant en composés de formule générale (III) 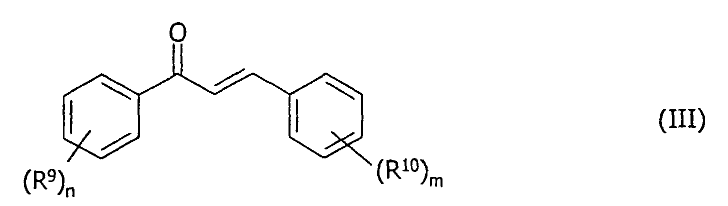 dans laquelle
n et m, identiques ou différents, sont égaux chacun à 0, 1, 2 ou 3, et
R⁹ et R¹⁰, ayant les significations identiques ou différentes, représentent chacun un groupe NR⁷R⁸, NO₂, OR⁷, alkyle en C₁-C₁₂, alcényle en C₂-C₁₂, alcynyle en C₂-C₁₂, cycloalkyle en C₅-C₈, aryle en C₆-C₁₂ ou hétéroaryle en C₁-C₁₂, chacun d'eux pouvant porter un ou plusieurs substituants halogénés identiques ou différents, et R⁷ et R⁸ ayant les significations indiquées ci-dessus en référence à la formule générale (II).

12. Procédé selon une ou plusieurs des revendications 1 à 11, **caractérisé en ce que** les homo-polyaminoacides enrichis en diastéréoisomères et énantiomères sont choisis dans le groupe consistant en la polynéopentylglycine, la polyleucine, la polyisoleucine, la polyvaline, la polyalanine et la polyphénylalanine.

13. Procédé selon une ou plusieurs des revendications 1 à 12, **caractérisé en ce que** la longueur de chaîne des polyaminoacides se situe dans l'intervalle de 5 à 100, de préférence de 7 à 50 et plus spécialement de 10 à 40 motifs répétés d'aminoacides.

14. Procédé selon une ou plusieurs des revendications 1 à 13, **caractérisé en ce que**, pour l'activation préalable de l'homopolyaminoacide, on met celui-ci en suspension avec le catalyseur à transfert de phase et en présence de la base et de l'agent oxydant dans un solvant organique et on agite pendant une durée de 15 min à 2 h après quoi, on isole l'homo-polyaminoacide ou bien, plus avantageusement, et après addition de l'énone α,β-insaturée ou de la sulfone α,β-insaturée, on utilise directement le mélange de réaction pour l'époxydation.

15. Procédé selon une ou plusieurs des revendications 1 à 14, **caractérisé en ce que** l'homo-polyaminoacide est mis en oeuvre en quantité de 0,1 à 40 mol%, de préférence de 0,5 à 20 mol% et plus spécialement de 1 à 15 mol% dans tous les cas par rapport à l'énone α,β-insaturée ou à la sulfone α,β-insaturée mise en oeuvre.

16. Procédé selon une ou plusieurs des revendications 1 à 15, **caractérisé en ce que** les agents oxydants utilisés sont des peroxydes, des peracides ou des agents oxydants minéraux comme l'hypochlorite de sodium ou le percarbonate de sodium.

17. Procédé selon une ou plusieurs des revendications 1 à 16, **caractérisé en ce que** l'agent oxydant utilisé est une solution aqueuse d'H₂O₂.

18. Procédé selon une ou plusieurs des revendications 1 à 17, **caractérisé en ce que** l'agent oxydant est mis en oeuvre en quantité de 1 à 40 équivalents, de préférence de 1 à 10 équivalents, plus spécialement de 1 à 3 équivalents et dans les meilleures conditions de 1,1 à 2,5 équivalents.

19. Procédé selon une ou plusieurs des revendications 1 à 18, **caractérisé en ce que** la base soluble dans l'eau mise en oeuvre est un hydroxyde de métal alcalin, de préférence NaOH, KOH ou LiOH.

20. Procédé selon une ou plusieurs des revendications 1 à 19, **caractérisé en ce que en ce que** la base est mise en oeuvre en quantité de 0,1 à 10 équivalents, de préférence de 0,5 à 5 équivalents et plus spécialement de 0,8 à 2 équivalents.

21. Procédé selon une ou plusieurs des revendications 1 à 20, **caractérisé en ce que** le solvant organique utilisé est un hydrocarbure aromatique substitué ou non, de préférence le toluène ou le xylène, un hydrocarbure aliphatique, de préférence l'hexane, un halogénoalcane, de préférence le chloroforme ou le chlorure de méthylène, ou un éther, de préférence l'oxyde de méthyle et de tert-butyle ou l'éther diéthylique.

22. Procédé selon une ou plusieurs des revendications 1 à 21, **caractérisé en ce que** la température de réaction se situe dans l'intervalle de -10 à +50°C, de préférence de 0 à +40°C et plus spécialement de +10 à +30°C.
